# EUROPEAN PATENT APPLICATION

(11) **EP 4 800 723 A1**
(43) Date of publication of application: **02.09.2026**
(21) Application number: 24890662.0
(22) Date of filing: 12.11.2024
(51) Int. Cl.: H01F 6/04

(54) **SUPERCONDUCTING MAGNET AND MAGNETIC RESONANCE IMAGING DEVICE**

(30) Priority: 15.11.2023 CN 202311536707
(71) Applicant: Shenzhen United Imaging Research Institute of Innovative Medical Equipment Innovation Research, Shenzhen, Guangdong 518045 (CN)
(72) Inventor: TONG, Junda, Shenzhen, Guangdong 518045 (CN)
(74) Representative: Murgitroyd & Company
(86) International application number: PCT/CN2024/131569
(87) International publication number: WO 2025/103307

(57) **Abstract**

A superconducting magnet includes a superconducting coil and a cooling mechanism. The superconducting coil includes a coil body and a heat exchange tube. The heat exchange tube is mounted on the coil body and is configured to exchange heat with the coil body. The cooling mechanism includes a cryocooler and a gas storage tank. The gas storage tank is in communication with the heat exchange tube.

## Description

This application claims priority to Chinese patent application No. 202311536707.X, entitled "Superconducting Magnet and Magnetic Resonance Imaging Device", filed with the Chinese Patent Office on November 15, 2023, the entire content of which is incorporated herein by reference.

### TECHNICAL FIELD

The present disclosure relates to the technical field of superconducting coil cooling, and in particular, to a superconducting magnet and a magnetic resonance imaging device.

### BACKGROUND

A superconducting coil is a core component of a superconducting magnet. A gas-liquid two-phase system of helium is used as a heat transfer medium for the superconducting coil. Under one atmosphere, a temperature of the superconducting coil is stabilized at 4.2 K to reach a superconducting state. As such, a high-stability, high-field-strength magnetic field can be generated when the superconducting coil operates to meet a use requirement of magnetic resonance scanning.

Currently, conventional superconducting magnets typically employ a manner of immersing a superconducting coil in liquid helium, and a heat exchange between the liquid helium and the superconducting coil is utilized, such that a temperature of the superconducting coil is stabilized at 4.2 K to reach a superconducting state. To this end, a superconducting magnet needs to be configured with a liquid helium chamber for accommodating liquid helium, and a large amount of liquid helium is injected into the liquid helium chamber to control an environmental temperature of the superconducting coil, such that a high-stability, high-field-strength magnetic field can be generated when the superconducting coil operates. However, to meet the requirement of immersing the superconducting coil in liquid helium, the superconducting magnet typically needs to be configured with hundreds to thousands of liters of liquid helium. As a non-renewable scarce resource, liquid helium is relatively expensive, which results in a high overall production cost of the superconducting magnet.

### SUMMARY

In an aspect of the present disclosure, a superconducting magnet is provided. The superconducting magnet includes:
a superconducting coil, including a coil body and a heat exchange tube, where the heat exchange tube is mounted on the coil body and is configured to exchange heat with the coil body; and
a cooling mechanism, including a cryocooler and a gas storage tank, where the gas storage tank is in communication with the heat exchange tube.

In some embodiments, the cooling mechanism includes a phase separator. The phase separator is connected to and in communication with the gas storage tank and the heat exchange tube, such that the gas storage tank is in communication with the heat exchange tube through the phase separator. The cryocooler has a second-stage cold head. The phase separator is mounted on the second-stage cold head and is configured to exchange heat with the second-stage cold head.

In some embodiments, the superconducting magnet further includes a room-temperature outer container. The room-temperature outer container defines a vacuum cavity therein. The phase separator and the superconducting coil are both accommodated in the vacuum cavity.

In some embodiments, the cryocooler further has a first-stage cold head. The gas storage tank is mounted on the first-stage cold head and is configured to exchange heat with the first-stage cold head.

In some embodiments, the first-stage cold head is configured to cool helium gas in the gas storage tank, and the second-stage cold head is configured to liquefy the helium gas that enters the phase separator after being cooled by the first-stage cold head. In some embodiments, the first-stage cold head is configured to provide a first cooling power in a first temperature range, and the second-stage cold head is configured to provide a second cooling power in a second temperature range. The first cooling power is greater than the second cooling power, and the first temperature range is higher than the second temperature range.

In some embodiments, the first temperature range is from 30 K to 300 K.

In some embodiments, the superconducting magnet further includes an external refrigerator. The external refrigerator is connected to and in communication with the gas storage tank, and is configured to exchange heat with the gas storage tank.

In some embodiments, a gas valve is provided in a path connecting the external refrigerator and the gas storage tank, and the gas valve is configured to control on/off of the path.

In some embodiments, the superconducting magnet further includes a cold shield. The cold shield is disposed around the superconducting coil and is configured to exchange heat with the gas storage tank.

In some embodiments, heat is exchanged between the cold shield and the gas storage tank through a flexible connecting strap.

In some embodiments, the superconducting magnet further includes a pipe located outside the cold shield, and two ends of the pipe are in communication with the gas storage tank.

In some embodiments, the pipe is provided with at least one additional gas storage tank in communication with the pipe, and the at least one additional gas storage tank is configured to exchange heat with the cold shield.

In some embodiments, the gas storage tank is connected to and in communication with a gas inlet pipe for introducing a refrigerant into the gas storage tank and a gas outlet pipe for discharging the refrigerant.

In some embodiments, the refrigerant includes liquid nitrogen.

In some embodiments, the superconducting magnet further includes a detection mechanism, and the detection mechanism is configured to monitor the liquid nitrogen discharged from the gas outlet pipe.

In some embodiments, the gas storage tank is configured to discharge the liquid nitrogen stored therein from the gas outlet pipe, when helium gas is introduced through the gas inlet pipe.

In some embodiments, the superconducting magnet further includes a room-temperature outer container. The room-temperature outer container defines a vacuum cavity therein. The gas storage tank is accommodated in the vacuum cavity.

In some embodiments, the first-stage cold head is mounted inside the gas storage tank, and/or the second-stage cold head is mounted inside the phase separator.

In another aspect of the present disclosure, a magnetic resonance imaging device is provided. The magnetic resonance imaging device includes the superconducting magnet according to any one of the above embodiments.

The details of various embodiments of the present disclosure will be described in the drawings and the description below. Other features, problems solved, and beneficial effects of the present disclosure will be readily understood by those skilled in the art from the description, the drawings, and the claims.

### BRIEF DESCRIPTION OF THE DRAWINGS

To more clearly describe the technical solutions in the embodiments of the present disclosure or in the conventional technology, the drawings to be used in the description of the embodiments or the conventional technology will be briefly introduced below. It is apparent that the drawings in the following description are merely some embodiments of the present disclosure. For those of ordinary skill in the art, other drawings can also be obtained based on these drawings without creative effort.
FIG. 1 is a schematic structural diagram of a superconducting magnet according to an embodiment of the present disclosure.
FIG. 2 is a schematic structural diagram of a superconducting magnet according to an embodiment of the present disclosure, where a gas storage tank is connected to and in communication with an external refrigerator.
FIG. 3 is a schematic structural diagram of a superconducting magnet according to an embodiment of the present disclosure, where liquid nitrogen can be first introduced into a gas storage tank through a gas inlet pipe.
FIG. 4 is a schematic structural diagram of a superconducting magnet according to another embodiment of the present disclosure.
FIG. 5 is a schematic structural diagram of a superconducting magnet according to another embodiment of the present disclosure.

### DETAILED DESCRIPTION

The technical solutions in the embodiments of the present disclosure will be clearly and completely described below in conjunction with the drawings in the embodiments of the present disclosure. Apparently, the described embodiments are merely some, rather than all, embodiments of the present disclosure. All other embodiments obtained by a person of ordinary skill in the art without inventive efforts based on the embodiments of the present disclosure shall fall within the scope of protection of the present disclosure.

It is noted that, when an element is referred to as being "disposed on" another element, the element may be directly disposed on the other element, or there may be an intervening element therebetween. When an element is considered to be "disposed on" another element, the element may be directly disposed on the other element, or there may be an intervening element therebetween at the same time. When an element is considered to be "fixed to" another element, the element may be directly fixed to the other element, or there may be an intervening element therebetween at the same time.

Unless otherwise defined, all technical and scientific terms used herein have the same meaning as commonly understood by a person skilled in the art to which the present disclosure belongs. The terms used in the description of the present disclosure are only for the purpose of describing specific embodiments, and are not intended to limit the present disclosure. The term "and/or" used herein includes any and all combinations of one or more of the associated listed items.

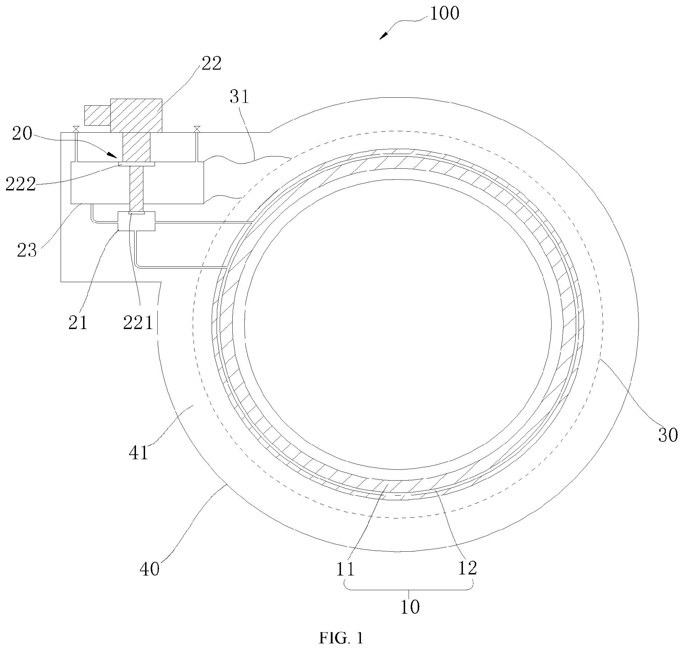

As shown in FIG. 1 to FIG. 5, a superconducting magnet 100 provided in some embodiments of the present disclosure includes a superconducting coil 10 and a cooling mechanism 20. The superconducting coil 10 includes a coil body 11 and a heat exchange tube 12. The heat exchange tube 12 is mounted on the coil body 11 and can exchange heat with the coil body 11. The cooling mechanism 20 includes a cryocooler 22 and a gas storage tank 23. The gas storage tank 23 is in communication with the heat exchange tube 12, such that a cooling gas or liquid in the gas storage tank 23 can enter the heat exchange tube 12 to exchange heat with the coil body 11.

In some embodiments, the cooling mechanism 20 includes a phase separator 21. The phase separator 21 is connected to and in communication with the gas storage tank 23 and the heat exchange tube 12, that is, the gas storage tank 23 is in communication with the heat exchange tube 12 through the phase separator 22. The phase separator 21 is configured to allow helium gas to enter therein for liquefaction and to supply liquid helium to the heat exchange tube 12. The cryocooler 22 has a second-stage cold head 221. The phase separator 21 is mounted on the second-stage cold head 221 and can exchange heat with the second-stage cold head 221. For example, the phase separator 21 may be mounted on the second-stage cold head 221 by welding. As shown in FIG. 1 to FIG. 3, in some embodiments, the second-stage cold head 221 is mounted inside the phase separator 21.

It can be understood that, in other embodiments, the second-stage cold head 221 is mounted on a surface of the phase separator 21. The second-stage cold head 221 can cool a helium medium in the phase separator 21, such that the helium medium is liquefied and flows into the heat exchange tube 12. That is to say, in the superconducting magnet 100 of the present disclosure, the heat exchange between the heat exchange tube 12 conducting liquid helium and the coil body 11 is utilized to lower a temperature of the coil body 11, such that the superconducting coil 10 reaches a superconducting state. It is noted that the cooling of the helium medium in the phase separator 21 by the second-stage cold head 221, such that the helium medium is liquefied and flows into the heat exchange tube 12, specifically means that the second-stage cold head 221 of the cryocooler 22 can liquefy gaseous helium gas in the phase separator 21 into liquid helium. At the same time, the second-stage cold head 221 can also cool the liquid helium in the phase separator 21. That is, the helium medium may include the helium gas and the liquid helium, where the helium gas may be liquefied into the liquid helium by the cooling of the phase separator 21 by the second-stage cold head 221.

It can be understood that the heat exchange between the heat exchange tube 12 conducting liquid helium and the coil body 11 is utilized to enable the superconducting coil 10 to reach the superconducting state, such that an amount of liquid helium used in the superconducting magnet 100 can be reduced, which can achieve effects of saving liquid helium resources and reducing an overall production cost of the superconducting magnet 100.

It is noted that the heat exchange tube 12 in the superconducting coil 10 of the present disclosure is arranged corresponding to the coil body 11. To improve a heat exchange efficiency in heat conduction between the heat exchange tube 12 and the coil body 11, a plurality of heat exchange fins may be arranged on the heat exchange tube 12. The plurality of heat exchange fins are respectively in contact engagement with the coil body 11, so as to increase a contact area in the heat exchange between the heat exchange tube 12 and the coil body 11. A specific arrangement of the heat exchange tube 12 on the coil body 11 may be configured according to a use requirement of the heat exchange between the heat exchange tube 12 and the coil body 11. For example, in some embodiments, the heat exchange tube 12 extends through an inside of the coil body 11, for example, in a helical shape. In other embodiments, the heat exchange tube 12 may be sleeved on an outside of the coil body 11.

As shown in FIG. 1 to FIG. 3, in some embodiments, the cooling mechanism 20 further includes the gas storage tank 23. The gas storage tank 23 is connected to and in communication with the phase separator 21, where the gas storage tank 23 can store helium gas, such that, when the cooling mechanism 20 operates, the gas storage tank 23 can supply helium gas to the phase separator 21, so as to enable the cryocooler 22 to liquefy the helium gas by cooling the phase separator 21 through the second-stage cold head 221, such that liquid helium can be conducted between the phase separator 21 and the heat exchange tube 12. The gas storage tank 23 can be configured to accommodate helium gas vaporized from liquid helium in the phase separator 21. For example, the gas storage tank 23 can accommodate the helium gas vaporized from the liquid helium in the phase separator 21. In some embodiments, both the gas storage tank 23 and the phase separator 21 may be configured as a tank structure.

As shown in FIG. 1 to FIG. 3, in some embodiments, the cryocooler 22 further has a first-stage cold head 222. The first-stage cold head 222 is mounted on the gas storage tank 23 and can exchange heat with the gas storage tank 23. For example, the gas storage tank 23 may be mounted on the first-stage cold head 222 by welding. As shown in FIG. 1 to FIG. 3, in some embodiments, the first-stage cold head 222 is mounted inside the gas storage tank 23. It can be understood that, in other embodiments, the first-stage cold head 222 is mounted on a surface of the gas storage tank 23. That is to say, when the cryocooler 22 operates, the helium gas in the gas storage tank 23 can be cooled by the first-stage cold head 222. As such, a high-power advantage of the first-stage cold head 222 of the cryocooler 22 can be utilized to pre-cool the helium gas, so as to facilitate subsequent liquefaction of the helium gas by the second-stage cold head 221 by cooling the phase separator 21. In this way, characteristics of the cryocooler 22 are fully utilized, and a cooling capacity of the cryocooler 22 is transferred to the coil body 11, such that the coil body 11 can maintain the superconducting state in a sufficiently low-temperature environment.

The first-stage cold head 222 is configured to provide a first cooling power in a first temperature range, and the second-stage cold head 221 is configured to provide a second cooling power in a second temperature range. The first cooling power is greater than the second cooling power, and the first temperature range is higher than the second temperature range. In some embodiments, the first-stage cold head 222 of the cryocooler 22 can provide a cooling power of tens to hundreds of watts in a temperature range of 30 K to 300 K. In some embodiments, the first-stage cold head 222 of the cryocooler 22 of the present disclosure is configured to, for example, maintain cooling in a temperature range of approximately 50 K. The second-stage cold head 221 is configured to, for example, mainly maintain cooling in a temperature range of approximately 4 K, and the cooling power of the second-stage cold head 221 is, for example, only in a range of one watt to several watts. The temperature of the coil body 11 needs to be stabilized at 4.2 K to reach the superconducting state. Therefore, in order for the coil body 11 to reach the superconducting state, the coil body 11 needs to maintain heat exchange with the second-stage cold head 221. It can be understood that, in other embodiments, working characteristics of the first-stage cold head 222 and the second-stage cold head 221 may be configured according to a temperature requirement for achieving superconductivity and a requirement for rapidly cooling the superconducting coil 10, so as to provide a suitable cooling power within a predetermined temperature range. The first-stage cold head 222 rapidly cools the helium gas in the gas storage tank 23 based on its high power, and then the cooled helium gas enters the gas storage tank 23. The helium gas is liquefied by the second-stage cold head 221 with low power, so as to transfer a cooling capacity to the coil body 11, such that the coil body 11 can maintain the superconducting state in the sufficiently low-temperature environment.

A specific structure of the cryocooler 22 and a working principle of how the first-stage cold head 222 and the second-stage cold head 221 are cooled during operation may both adopt a conventional manner of a cryocooler currently applied in magnetic resonance imaging technology, and details are not described herein.

As shown in FIG. 1 to FIG. 3, in some embodiments, the superconducting magnet 100 further includes a cold shield 30. The cold shield 30 is disposed around the superconducting coil 10. The gas storage tank 23 is configured to exchange heat with the cold shield 30. For example, heat can be exchanged between the cold shield 30 and the gas storage tank 23 through a flexible connecting strap 31. As such, the gas storage tank 23 can be used to cool the cold shield 30, and structural characteristics of the cold shield 30 can be utilized to reduce radiation heat received by the superconducting coil 10, such that an environmental temperature of the coil body 11 can be prevented from being affected by external thermal radiation.

It is noted that specific structures of the cold shield 30 and the flexible connecting strap 31, a manner in which heat is exchanged between the gas storage tank 23 and the cold shield 30 through the flexible connecting strap 31, and a working principle of how the cold shield 30 reduces radiation heat may all adopt a conventional manner of a cold shield currently applied in magnetic resonance imaging technology, and details are not described herein.

As shown in FIG. 1 to FIG. 3, in some embodiments, the superconducting magnet 100 further includes a room-temperature outer container 40. The room-temperature outer container 40 defines a vacuum cavity 41 therein. The phase separator 21, the gas storage tank 23, and the superconducting coil 10 are all accommodated in the vacuum cavity 41, such that a vacuum environment can be created for the superconducting coil 10. As such, convective heat transfer of the superconducting coil 10 inside the room-temperature outer container 40 can be blocked, such that an environmental temperature of the superconducting coil 10 can be prevented from being affected by convective heat transfer. It is noted that the room-temperature outer container 40 is specifically formed as an outer housing of the superconducting magnet 100, and the vacuum cavity 41 defined inside the room-temperature outer container 40 specifically means that a vacuum environment can be formed inside the room-temperature outer container 40. A vacuum degree of the vacuum cavity 41 may be specifically set according to a requirement, and details are not described herein.

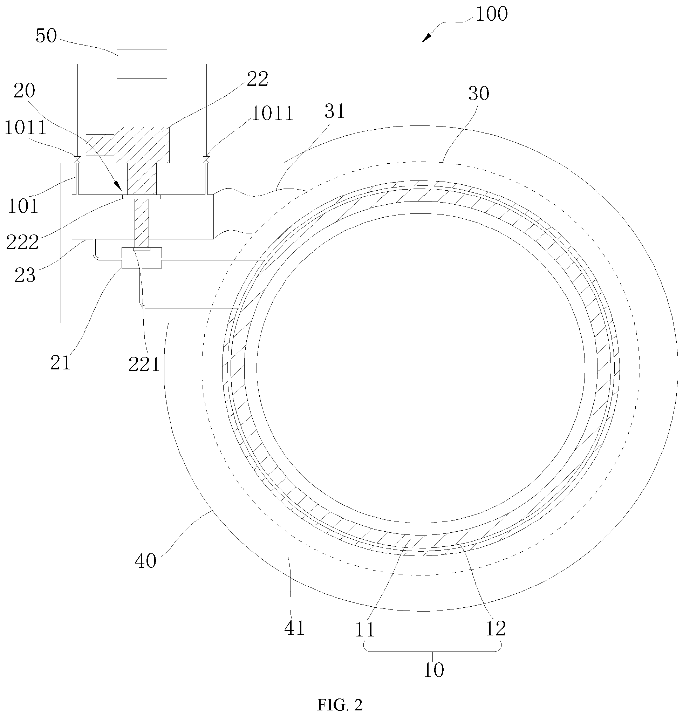

As shown in FIG. 2, in some embodiments, the superconducting magnet 100 further includes an external refrigerator 50. The external refrigerator 50 is connected to and in communication with the gas storage tank 23, where the external refrigerator 50 can cool the helium gas. That is to say, when the superconducting magnet 100 of this embodiment operates, the external refrigerator 50 can be utilized to rapidly cool the helium gas in the gas storage tank 23 so as to cool the coil body 11. After the coil body 11 reaches a specified temperature, the heat exchange between the external refrigerator 50 and the gas storage tank 23 is then disconnected. Liquefaction of the helium gas is achieved by cooling the phase separator 21 through the second-stage cold head 221, so as to ultimately maintain a thermosiphon system equilibrium of the liquid helium. As such, a cooling time for cooling the coil body 11 during operation of the superconducting magnet 100 can be greatly shortened. It is noted that the external refrigerator 50 may be configured as a refrigerator currently conventionally applied in magnetic resonance imaging technology according to a requirement, and details are not described herein.

In some embodiments, a gas valve 1011 is provided in a path 101 connecting the external refrigerator 50 and the gas storage tank 23, and the gas valve 1011 can control on/off of the path 101. That is to say, on or off between the external refrigerator 50 and the gas storage tank 23 can be achieved through the gas valve 1011. As such, an intervention of the cooling of the helium gas by the external refrigerator 50 can be controlled, so as to meet a use requirement of subsequently liquefying the helium gas into the liquid helium by the cryocooler 22. It is noted that the gas valve 1011 may be configured as a manual valve, a solenoid valve, etc., according to a requirement, and details are not described herein.

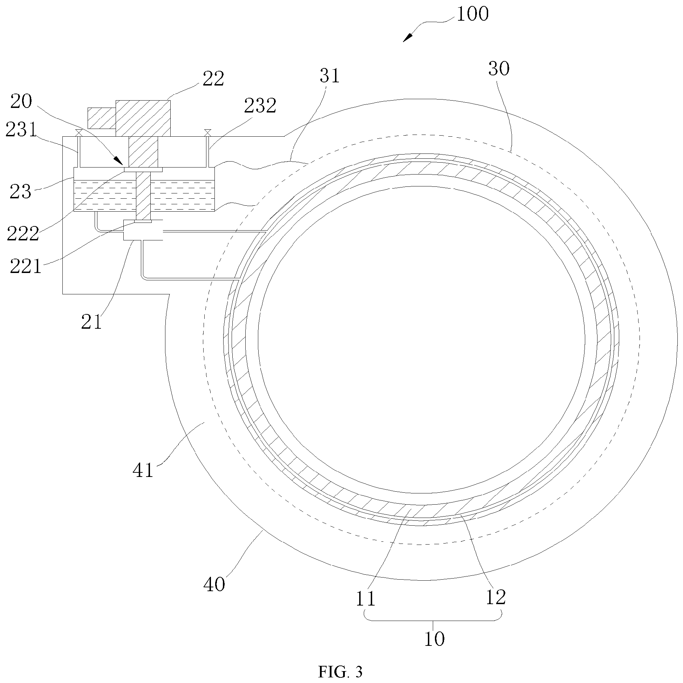

As shown in FIG. 3, in some embodiments, a gas inlet pipe 231 and a gas outlet pipe 232 are connected to and in communication with the gas storage tank 23. A refrigerant may be first introduced through the gas inlet pipe 231, and the refrigerant cools the gas storage tank 23 and the coil body 11. After thermal equilibrium is reached, helium gas is then introduced into the gas inlet pipe 231. By using a difference in specific gravity between the helium gas and the refrigerant, the refrigerant can be discharged out through the gas outlet pipe 232 until emptied, and then the gas inlet pipe 231 and the gas outlet pipe 232 are closed. For example, the refrigerant may include liquid nitrogen, liquid carbon dioxide, liquid propane, etc. Taking liquid nitrogen as an example, liquid nitrogen can be introduced into the gas storage tank 23 through the gas inlet pipe 231. After the introduced liquid nitrogen reaches thermal equilibrium, the introduced liquid nitrogen can be pushed by the helium gas introduced through the gas inlet pipe 231 and discharged out through the gas outlet pipe 232, such that the liquid nitrogen is emptied from the gas storage tank 23. That is to say, in a process of cooling the coil body 11 by the superconducting magnet 100 of this embodiment, liquid nitrogen may be first introduced through the gas inlet pipe 231, and the liquid nitrogen is utilized to cool the gas storage tank 23 and the coil body 11. After thermal equilibrium is reached, helium gas is then introduced into the gas inlet pipe 231. By using a difference in specific gravity between the helium gas and the liquid nitrogen, the liquid nitrogen can be discharged out through the gas outlet pipe 232 until emptied, and then the gas inlet pipe 231 and the gas outlet pipe 232 are closed. Thereafter, the cryocooler 22 is turned on, the gas storage tank 23 is further cooled by the first-stage cold head 222, and liquefaction of the helium gas is achieved by cooling the phase separator 21 through the second-stage cold head 221, so as to ultimately maintain the thermosiphon system equilibrium of the liquid helium. Since liquid nitrogen is inexpensive and easily available compared to helium gas, the above manner can not only greatly shorten a cooling time of the superconducting coil 10, but can also reduce a cost. It is noted that the above-mentioned thermal equilibrium of the liquid nitrogen in the gas storage tank 23 specifically means that a temperature of the gas storage tank 23 into which the liquid nitrogen is introduced is consistent with a temperature of the liquid nitrogen, and no further heat exchange occurs between the two.

It is noted that a temperature of liquid nitrogen is generally 77 K. After the cryocooler 22 is turned on, the first-stage cold head 222 can lower the helium gas in the gas storage tank 23 to 50 K, and the second-stage cold head 221 can cool the liquid helium in the phase separator 21 to 4 K. Therefore, in a process of introducing liquid nitrogen into the gas storage tank 23 through the gas inlet pipe 231 until the liquid nitrogen reaches thermal equilibrium, the cryocooler 22 does not need to be turned on for operation.

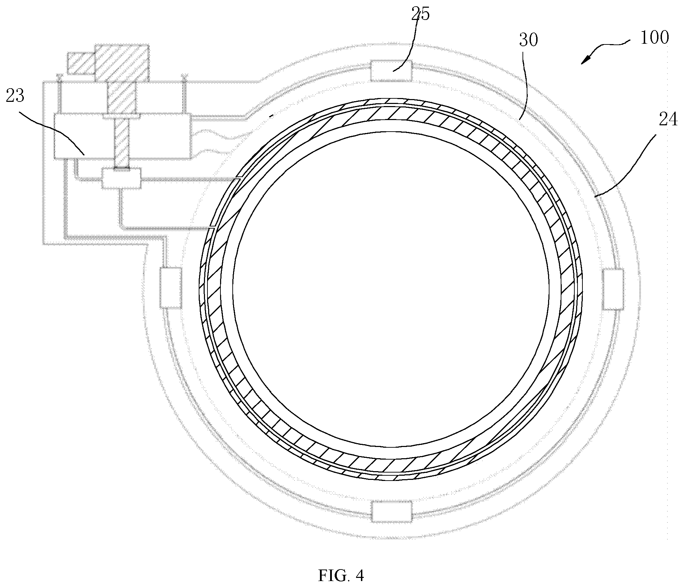

Referring to FIG. 4, in some embodiments, the superconducting magnet 100 further includes a pipe 24 located outside the cold shield 30, and two ends of the pipe 24 are in communication with the gas storage tank 23. The helium gas in the gas storage tank 23 enters the pipe 24, such that the helium gas can exchange heat with the cold shield 30. The pipe 24 is further provided with at least one additional gas storage tank 25 that is in communication with the pipe 24 and is used to store helium gas. As shown in FIG. 4, the pipe 24 is provided with a plurality of additional gas storage tanks 25 connected in series. The plurality of additional gas storage tanks 25 are in communication with the pipe 24, such that, on the one hand, a gas storage capacity can be increased, and on the other hand, the plurality of additional gas storage tanks 25 can be in contact with the cold shield 30 so as to further exchange heat with the cold shield 30.

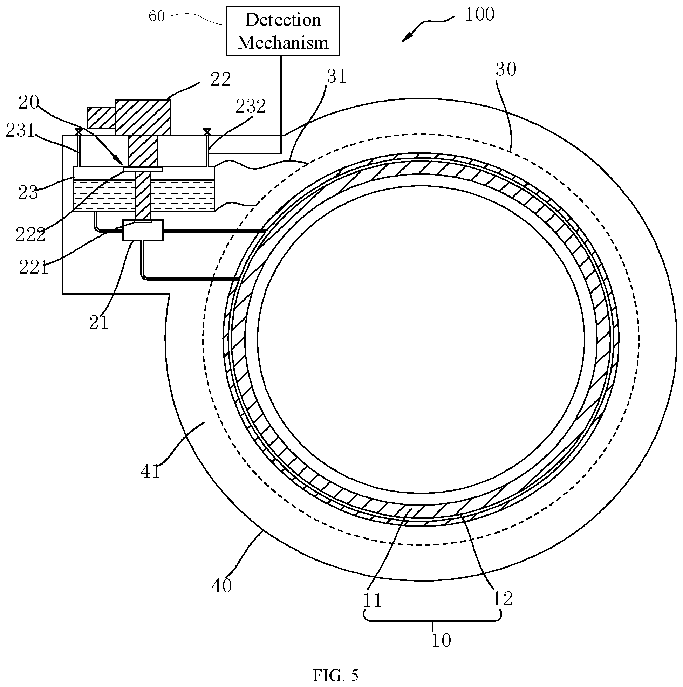

In some embodiments, referring to FIG. 5, the superconducting magnet 100 further includes a detection mechanism 60. The detection mechanism 60 can monitor discharge of the liquid nitrogen in the gas storage tank 23, so as to determine that the liquid nitrogen in the gas storage tank 23 has been emptied, such that the superconducting magnet 100 can wait until the liquid nitrogen in the gas storage tank 23 is completely emptied, and then control the cryocooler 22 to be turned on.

For example, the detection mechanism 60 can specifically determine that the liquid nitrogen in the gas storage tank 23 has been emptied by determining whether helium gas is mixed in the liquid nitrogen discharged from the gas outlet pipe 232. Alternatively, a weight comparison of the superconducting magnet 100 before and after the liquid nitrogen is introduced may be utilized, and whether the liquid nitrogen in the gas storage tank 23 has been emptied may be determined by weighing the superconducting magnet 100.

In some embodiments, the path 101 connecting the external refrigerator 50 and the gas storage tank 23, the gas inlet pipe 231, and the gas outlet pipe 232 may share a pipeline. In other embodiments, the path 101 connecting the external refrigerator 50 and the gas storage tank 23, the gas inlet pipe 231, and the gas outlet pipe 232 may be independent of one another.

In addition, the present disclosure further provides a magnetic resonance imaging device. The magnetic resonance imaging device includes the superconducting magnet 100 according to the above embodiments.

In summary, in the superconducting magnet 100 claimed in the present disclosure, the heat exchange between the heat exchange tube 12 and the coil body 11 is utilized to lower an environmental temperature of the coil body 11, such that the superconducting coil 10 reaches the superconducting state. As such, an amount of liquid helium used in the superconducting magnet 100 can be reduced, which can achieve effects of saving liquid helium resources and reducing an overall production cost of the superconducting magnet 100.

The technical features of the above embodiments may be combined arbitrarily. For brevity of description, not all possible combinations of the various technical features in the above embodiments are described. However, all combinations of these technical features shall be considered as falling within the scope described in this specification, as long as no contradiction exists in such combinations.

It will be appreciated by those of ordinary skill in the art that the foregoing embodiments are merely used to describe the present disclosure and are not intended to limit the present disclosure. Any appropriate modifications and changes to the foregoing embodiments fall within the scope of protection claimed by the present disclosure as long as they are within the substantive spirit of the present disclosure.

## Claims

1. A superconducting magnet, comprising:
a superconducting coil (10), comprising a coil body (11) and a heat exchange tube (12), wherein the heat exchange tube (12) is mounted on the coil body (11) and configured to exchange heat with the coil body (11); and
a cooling mechanism (20), comprising a cryocooler (22) and a gas storage tank (23), wherein the gas storage tank (23) is in communication with the heat exchange tube (12).

2. The superconducting magnet according to claim 1, wherein the cooling mechanism (20) comprises a phase separator (21), the phase separator (21) is connected to and in communication with the gas storage tank (23) and the heat exchange tube (12), such that the gas storage tank (23) is in communication with the heat exchange tube (12) through the phase separator (21), the cryocooler (22) has a second-stage cold head (221), and the phase separator (21) is mounted on the second-stage cold head (221) and configured to exchange heat with the second-stage cold head (221).

3. The superconducting magnet according to claim 2, wherein the superconducting magnet (100) further comprises a room-temperature outer container (40), the room-temperature outer container (40) defines a vacuum cavity (41) therein, and the phase separator (21) and the superconducting coil (10) are both accommodated in the vacuum cavity (41).

4. The superconducting magnet according to any one of claims 1 to 3, wherein the cryocooler (22) further has a first-stage cold head (222), and the gas storage tank (23) is mounted on the first-stage cold head (222) and configured to exchange heat with the first-stage cold head (222).

5. The superconducting magnet according to claim 4, wherein the first-stage cold head (222) is configured to cool helium gas in the gas storage tank (23), and the second-stage cold head (221) is configured to liquefy the helium gas that enters the phase separator (21) after being cooled by the first-stage cold head (222).

6. The superconducting magnet according to claim 5, wherein the first-stage cold head (222) is configured to provide a first cooling power in a first temperature range, the second-stage cold head (221) is configured to provide a second cooling power in a second temperature range, the first cooling power is greater than the second cooling power, and the first temperature range is higher than the second temperature range.

7. The superconducting magnet according to claim 6, wherein the first temperature range is from 30 K to 300 K.

8. The superconducting magnet according to any one of claims 1 to 7, wherein the superconducting magnet (100) further comprises an external refrigerator (50), the external refrigerator (50) is connected to and in communication with the gas storage tank (23), and the external refrigerator (50) is configured to exchange heat with the gas storage tank (23).

9. The superconducting magnet according to claim 8, wherein a gas valve (1011) is provided in a path (101) connecting the external refrigerator (50) and the gas storage tank (23), and the gas valve (1011) is configured to control on/off of the path (101).

10. The superconducting magnet according to any one of claims 1 to 9, wherein the superconducting magnet (100) further comprises a cold shield (30), the cold shield (30) is disposed around the superconducting coil (10), and the gas storage tank (23) is configured to exchange heat with the cold shield (30).

11. The superconducting magnet according to claim 10, wherein heat is exchanged between the cold shield (30) and the gas storage tank (23) through a flexible connecting strap (31).

12. The superconducting magnet according to claim 10 or 11, further comprising a pipe (24) located outside the cold shield (30), wherein two ends of the pipe (24) are in communication with the gas storage tank (23).

13. The superconducting magnet according to claim 12, wherein the pipe (24) is provided with at least one additional gas storage tank (25) in communication with the pipe (24), and the at least one additional gas storage tank (25) is configured to exchange heat with the cold shield (30).

14. The superconducting magnet according to any one of claims 1 to 13, wherein the gas storage tank (23) is connected to and in communication with a gas inlet pipe (231) for introducing a refrigerant into the gas storage tank (23) and a gas outlet pipe (232) for discharging the refrigerant.

15. The superconducting magnet according to claim 14, wherein the refrigerant comprises liquid nitrogen.

16. The superconducting magnet according to claim 15, wherein the superconducting magnet (100) further comprises a detection mechanism, and the detection mechanism is configured to monitor the liquid nitrogen discharged from the gas outlet pipe (232).

17. The superconducting magnet according to claim 15 or 16, wherein the gas storage tank (23) is configured to discharge the liquid nitrogen stored therein from the gas outlet pipe (232) when helium gas is introduced through the gas inlet pipe (231).

18. The superconducting magnet according to claim 1 or 2, wherein the superconducting magnet (100) further comprises a room-temperature outer container (40), the room-temperature outer container (40) defines a vacuum cavity (41) therein, and the gas storage tank (23) is accommodated in the vacuum cavity (41).

19. The superconducting magnet according to any one of claims 1 to 18, wherein the first-stage cold head (222) is mounted inside the gas storage tank (23), and/or the second-stage cold head (221) is mounted inside the phase separator (21).

20. A magnetic resonance imaging device, comprising the superconducting magnet (100) according to any one of claims 1 to 19.
